# EUROPEAN PATENT SPECIFICATION

(11) **EP 1 325 756 B1**
(45) Date of publication and mention of the grant of the patent: **09.02.2011**
(21) Application number: 02028711.6
(22) Date of filing: 19.12.2002
(51) Int. Cl.: A61L 29/14, A61L 15/00

(54) **Delivery source of oxygen and kit for producing a medical device**
Sauerstoffabgabequelle und Kit für die Herstellung einer medizinischen Vorrichtung
Source d'administration d'oxygene et kit pour la production d'un dispositif medicale

(30) Priority: 03.01.2002 US 38468
(43) Date of publication of application: 09.07.2003
(73) Proprietor: Hehrlein, Christoph, Dr., 79104 Freiburg (DE)
(72) Inventor: Hehrlein, Christoph, Dr., 79104 Freiburg (DE); Kovacs, Adalbert, Dr., 69214 Eppelheim (DE); Wolf, Gerhard Karl, Prof. Dr., 69120 Heidelberg (DE)
(74) Representative: Cullinane, Marietta Bettina

(56) References cited:
- EP-A- 0 112 658
- EP-A- 0 372 088
- US-A- 4 366 169

## Description

The invention relates to an oxygenating source of fluids and tissue for medical purposes and a fluorocarbon solution for the use in the medical field. The field of the invention is the treatment of ischemic disorders in diseases, for example in cardiovascular disease. The material disclosed herein improves healing of lacerated hypoxic tissues, ulcers and poorly vascularized wounds.

### Background of the invention

A percutaneous transluminal angioplasty (PTA) or bypass surgery of obstructed blood vessels is a very popular method to restore lack of oxygen supply of a human organ.

One of the principle limitations of a percutaneous transluminal angioplasty (PTA) is the complete obstruction of blood flow during the inflation of the balloon. Patients undergoing percutaneous coronary angioplasty (PTCA) experience myocardial ischemia due to the lack of flow of oxygenated blood to the myocardium after a short period of balloon occlusion. Angina pectoris resulting from myocardial ischemia is usually indicated by either changes in the electrocardiogram or cardiac arrhythmias. In the past, perfusion balloon catheters were developed to overcome the problem of blood flow obstruction during percutaneous coronary interventions. However, perfusion balloon catheters placed into small arteries such as the coronary circulation have the disadvantage of limited blood perfusion capacity inherent to relatively small blood flow rates in those arteries. This reduced perfusion capacity of a standard perfusion catheter reduces inflation time, and sometimes requires termination of the angioplasty with little gain. An adequate prolongation of the dilatation process, for instance to achieve better angioplasty results, may not be achieved with standard balloon perfusion catheters. The inflated angioplasty balloon often occludes the vasa vasorum of a conduit vessel, and is thereby also preventing oxygen supply to depending organs via connecting microvessels. Complicated PTCA procedures such as dilatation of the left main coronary artery require optimal oxygenation of the distal vasculature during balloon inflation.

Patients with severe occlusive arterial disease are frequently treated with bypass surgery using artificial polymer grafts. In patients with diabetes, however, revascularization via bypass grafting is often not sufficient to heal foot ulcers and gangrene. In some of these cases, amputation of the foot cannot be avoided. New oxygen treatments include placement of patients with diabetic foot ulcers into hyperbaric oxygen chambers, which has been shown to improve healing of skin wounds, and reduce amputation rates. However, these treatments are expensive and cumbersome for patients.

Oxygenated fluorocarbons emulsions injected into the vasculature have been used for years to treat hypoxic and ischemic disorders. Oxygen-transferable fluorocarbon emulsions became known as artificial blood substitutes more than twenty years ago. In US patent 3,958,014 and US patent 4,252,827, fluorocarbon emulsion are disclosed that have a very small particle size of 0.02 to 0.25 microns, which prevent embolic damage. In US patent 4,445,500, Osterholm teaches that oxygenated fluorocarbon emulsions can be injected into the cerebrospinal pathway to improve aerobic respiration of brain tissue. US patent 4,795,423 (Osterholm) discloses an intraocular perfusion with perfluorinated substances to treat ischemic retinopathy.

However, the local delivery of drugs using fluids injected into organs via needles or catheters for treatment purpose have disadvantages. US patent No. 4,636,195 teaches that liquid substances may be injected through a porous balloon catheter. The injection of substances into blood vessels may cause damage of vascular structures during the injection process, especially if those liquids are injected directly into tissue or propelled through pores of the wall of the balloon into the vessel wall. The direct needle injection of liquids into tissues can cause severe injury and death of the target cells by itself. The trauma of a vessel wall during injection may promote neointimal hyperplasia of the wall as a cause of stenosis. Even modified surfaces of infusions balloons with dimples as disclosed in US patent 6,048,332 may not completely prevent vascular injury during injection therapeutic agents at the time of balloon inflation. Another disadvantage of direct injection of therapeutic liquids into tissues is the lack of sustained local drug delivery.

EP 0 372 088 discloses a balloon catheter with oxygen delivery through pores but the oxygen carrier is introduced through the catheter.

An object of the present invention is therefore to provide means for supplying oxygen to parts of a human body, in particular to body fluids and tissue, without causing disadvantageous effects on the tissue and which at the same time allow a controllable delivery of oxygen to body fluids.

### Summary of the Invention

This invention consists of an oxygen delivery source that allows the local diffusion of a liquid oxygen carrier into bodily fluids and tissue as defined by claim 1, and is capable of oxygenating blood and tissue. One of the major goals of the invention is to improve sustained oxygen supply to ischemic organs, for instance during and after a revascularization procedure such as a balloon angioplasty or vascular bypass surgery. The liquid oxygen carrier may be released to the target area from a part of a catheter, from a tube, a perfusion balloon, an implant or a tissue patch. The invention therefore also relates to a medical device, which is at least partially formed by a membrane, which in use can serve as the substrate of a delivery source according to the invention. The features and advantages of the delivery source relate as far as they are applicable also to the medical device and vice versa. The invention presented here allows local and systematical diffusion of a liquid oxygen carrier into a hypoxic target tissue, in which oxygen is released from the carrier. The purpose of the invention is a localised sustained oxygen release during and after a medical procedure and to hypoxic tissue, in particular wounds.

A preferred embodiment is a medical device for insertion into a blood vessel comprising membrane-like porous structure or a polymer film, which is impregnated with a liquid oxygen carrier. Polymers such as teflon, polyethylen, polyethylene terephtalate, polyphosphazene, nylon, silicon, and cellulose actetate with small pore sizes of 20 - 200 microns are preferable substrates for effective incorporation of the oxygenated liquid oxygen carrier. The impregnation process may take place at the operation table bringing the surface of the source in contact with the liquid oxygen carrier. The source membrane may be dipped into the prepared solution of a liquid oxygen carrier such as an oxygenated fluorocarbon solution at the time of use. The substance for the liquid oxygen carrier is an oxygenated fluorocarbon solution. The use of a solution of an oxygen carrier has several advantages over the use of an emulsion of an oxygen carrier. The fluorocarbon solution is externally saturated with oxygen prior to incorporation into the substrate. Thus, two separately prepared components form a kit.
1. A source membrane and
2. A fluorocarbon solution saturated with oxygen.
   These components of the kit are brought together at the time of the use.

The fluorocarbon solution is preferably contained in a housing for transport and storage, wherein the housing is sealed to prevent leakage of gas and liquid. In particular the sealing prevents dissipation of oxygen. The housing may be a container made of glass or metal and may comprise a removable film coating or a plastic sheath. The kit according to the invention preferably further comprises a medical device, at least a part of which is formed by a membrane. The membrane may be attached to or included in the medical device. The medical device may be a hollow tube, a stent, a catheter, in particular a balloon catheter, e.g. a perfusion balloon catheter, a bypass graft an endovascular stent inserted temporarily or permanently into or at a blood vessel, a transvascular implant directly connected to blood vessels and / or a tissue patch for external treatment of wounds with oxygen.

The fluorocarbon solution used according to the invention is preferably artificially oxygenated. The degree of saturation of oxygen should be the optimally achievable degree and is preferably in the range of 80 to 100%, in particular 95-100%. The fluorocarbon solution can be artificially oxygenated and subsequently covered with a protective means providing a housing with a sealed system preventing leakage of oxygen out of the sealed system. Thereby the liquid oxygen carrier to be used for impregnation of the delivery source, e.g. medical devices, can be transported and stored separately from the source.

In another embodiment, a source membrane is pre-incubated or pre-impregnated with the liquid oxygen carrier and stored in a removable housing to prevent the escape of oxygen molecules and liquid. Polymers such as teflon, polyethylen, polyethylene terephtalate, polyphosphazen, nylon, silicon, and cellulose actetate with small pore sizes of 20 - 200 microns are preferable substrates for effective incorporation for oxygenated fluorocarbon solutions. The liquid oxygen carrier is released from the membrane via diffusion, in particular via the pores of the membrane, and thus minimises local injury of the vasculature. The release kinetics of the solution from the structure are modulated by controlled temperature changes of the environment. These temperature changes are induced, for instance, by application of cold and warm liquids around the device. Studies on the release kinetics of the oxygenated fluorocarbons from different polymer membranes show that elution of the oxygenated fluorocarbon solution from such a membrane into tissue or blood varies between minutes and several hours depending on the temperature of the environment. A protective housing for storing either the oxygen carrier alone or the assembly including the source, for example as part of or as a medical or surgical instrument and device, respectively, is made out of plastic, glass or metal to prevent the loss of gas and liquid.

One embodiment of this invention is a catheter based local oxygen delivery as a means for cardiovascular protection, which combines tissue oxygenation via diffusion release of an oxygenated liquid with a direct fluid injection into the blood stream. In this embodiment, the oxygen delivery substrate is located on the surface of a balloon of a perfusion angioplasty catheter. A porous polymer membrane at a thickness between 20 and 200 µm is tightly wrapped around the balloon, or is tightly attached to the balloon surface. At the operating table, the balloon surface is inflated and dipped into a container of a prepared solution of oxygenated fluorocarbons serving as the liquid oxygen carrier. The catheter with the membrane is advanced into the circulation, and after release of the oxygen carrier may be re-used after retrieval by repeating the dipping process. In another embodiment, the manufacturer provides an assembly of the medical device including a housing in which the liquid oxygen carrier is already attached to the surface. At the site of the surgical procedure, the substrate membrane is brought in contact with the target cell. In case of oxygen delivery via a perfusion balloon, the oxygen enters into the blood vessel wall by diffusion. A certain amount of oxygen also enters the blood directly from the membrane via diffusion. Injection of warm or cold fluids via the perfusion channel of the balloon is used to modify the timing of release of the liquid oxygen carrier to the blood vessel and the blood. Direct contact of the device with target tissues improves oxygen delivery, and does not cause tissue trauma. The capacity of oxygen saturation of end organ increases with the improvement of blood flow, which is maintained through perfusion of the balloon with blood.

Another embodiment of the invention is a permanent implant such as an impregnated stent or a bypass graft, which consist of or is coated with a membrane that stores and elutes the liquid oxygen carrier. Polymeric bypass grafts are particularly useful for oxygen release as presented herein because these grafts have huge surface areas for blood oxygenation. The graft is coated at the inside and / or outside with the substrate for the release of the oxygen carrier. A certain porosity of the basic polymer material of the substrate for carrier release is induced in the range of 20 to 200 microns. Alternatively, porous films may be firmly attached to the non-porous surfaces of the surgical instrument. It is disclosed herein that preferably microporous material is impregnated with a liquid oxygen carrier. Perfusion channels carrying liquids through and / or around the therapeutic device are designed to allowing controlled temperature changes in area of release of the oxygen carrier. The induced local temperature changes accelerate or slow down the escape of oxygen from the carrier membrane.

In another embodiment, multiple polymer membranes are rolled together as tubes and are impregnated with the liquid oxygen carrier at their inner surface. A multitude of these tubes are assembled together to form an implant oxygenator. Preferably the oxygenator comprises one inner tube and one outer tube, which are coaxially and concentrically arranged to each other. In the spacing formed between these two tubes further tubes may be provided in a coaxial fashion. In this embodiment the inner tube and the outer tube may be impregnated with the liquid oxygen carrier on the inside and the outside respectively. The tubes provided in the spacing between the inner and outer tube may serve as channels for cold or warm fluid. The inner tube may serve as a blood channel.

The implant oxygenator is connected by an end-to-end or end-to-side-anastomosis with a supplying blood vessel and a receiving blood vessel. The blood is carried through the tubes impregnated with the liquid oxygen carrier. This process may be used to supplement oxygen delivery to the blood during a cardiopulmonary bypass procedure.

In another embodiment, an oxygenated tube assembly as disclosed previously, may serve as a temporarily placed membrane-based oxygenator in a cavity of the human body. The polymer based membrane oxygenator may be, for instance, connected to ventricles of the brain for oxygenation of the cerebrospinal fluid.

Modified stent delivery balloons, i.e. balloon catheters with a pre-mounted stent or perfusion balloons are one of the embodiments of the invention. Endovascular stents themselves may be coated with a thin film membrane incorporating the liquid oxygen carrier. The stents are permanent implants and provide a sustained release of oxygen. For prevention of restenosis, local delivery of oxygenated fluorocarbon solution may be combined with the application of ionizing radiation or low energy ultraviolet light to increases the production of oxygen free radicals in the target cell. The effect of increased oxygen free radical production of the proliferating target cell in the arterial wall is DNA damage, which will reduce restenosis formation.

Several other clinical applications, not being a part of the claimed invention, related to the field of vascular medecine are suggestive for a therapeutic device that provides local tissue oxygenation. Wound

healing of skin lacerations in patients with peripheral occlusive arterial disease and impaired blood flow in the lower limb organs may be significantly improved with the local delivery of an oxygenated fluorocarbon solution via a skin patch placed onto the ischemic skin. In another embodiment of said invention, wounds are covered with flaps of membranes impregnated with the oxygen carrier. First, the wound is cleaned, and necrotic tissue is removed. Thereafter, a piece of the substrate membrane impregnated with a liquid oxygen carrier is externally attached to the wound of the skin. The patch is removed after the oxygen has completely diffused into the lacerated tissue. The local increase in oxygen tension promotes growth of new blood vessels into the area of ischemia. Gangrenes of the lower limb due to oxygen deficits will be reduced in size.

In another embodiment, the oxygen carrier release from the substrate membrane is accompanied by a co-release of a pharmologically active agent from the membrane. The co-release of the pharmologically active agent may be performed by injection close to the membrane and / or by diffusion from the membrane. This co-released agent may have anti-proliferative, vasodilatative, or anti-oxidant properties. The purpose of the co-release of vasoactive agents is to increase diffusion areas for the oxygen in tissue, and accelerate transport of liquid oxygen through the vasculature.

### Brief Description of the Drawings

Fig. 1 shows a schematic perspective view of a microporous thin film membrane being part of a medical device. The membrane functions as the flexible substrate for the liquid oxygen carrier to be store and released in accordance with the invention disclosed herein.
Fig. 2 shows a schematic perspective of a perfusion balloon catheter covered with a thin film membrane for oxygen delivery in accordance with the invention disclosed herein.
Fig. 3 schematic cross-sectional view of a medical device containing a liquid oxygen delivery source being encompassed by a housing sealing off the source in accordance with the present invention.

### Detailed Description of the Drawings

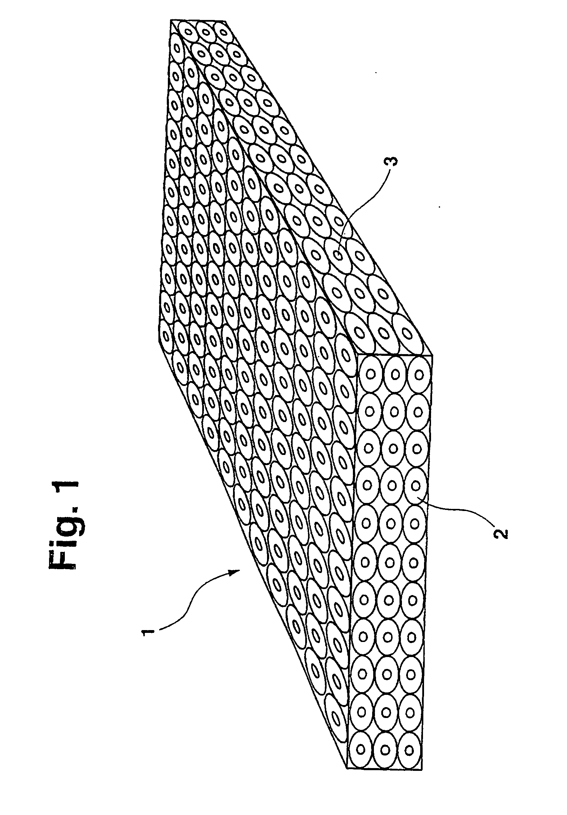

Figure 1 shows a perspective view of a porous thin film membrane 1 with pores 2 functioning as a flexible substrate for a liquid oxygen carrier 3. An oxygenated fluorocarbon solution is the substance for incorporation and elution from the substrate. The liquid oxygen carrier diffuses freely out of the thin film membrane 1, in particular out of the pores 2. The temperature-dependent release feature of the membrane 1 can be used in all medical devices described herein. Generally, the release kinetics from the substrate may be controlled by injection of fluids 0-50° Celsius which make direct or indirect contact with the substrate incorporating the oxygen carrier.

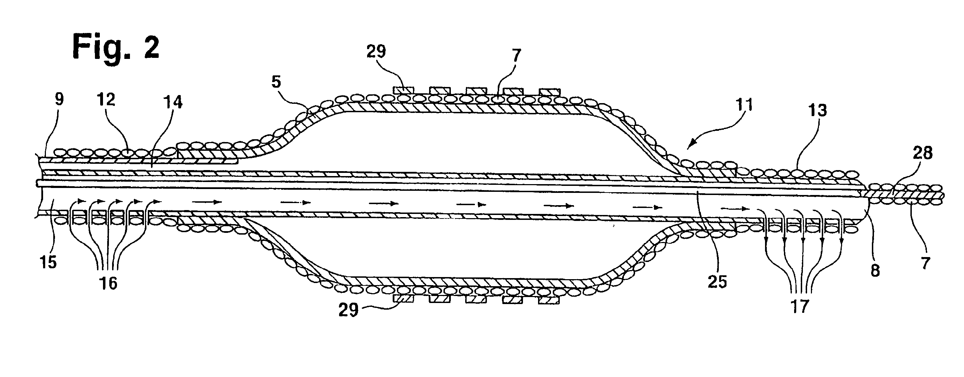

Figure 2 shows a schematic longitudinal view of an perfusion balloon catheter 11 serving as the substrate source for the liquid oxygen carrier. In this embodiment, an oxygen delivery source membrane 7 is located on the surface of the balloon 5 and proximally 12 and distally 13 to the balloon end of the catheter 11 on the shaft 9 of the catheter. The shaft 9 of the perfusion balloon catheter includes the guide wire lumen 8, a balloon inflation lumen 14, and a perfusion lumen 15. The perfusion fluid lumen allows perfusion of blood or transport of therapeutic fluids (temperature between 0-50°C) through the inflated balloon. The perfusion lumen 15 is designed to allow injection of therapeutic liquids or drugs with temperatures between 0 and 50° C to modify the release kinetics of the oxygen carrier from the substrate. Holes beyond the proximal end 16 of the balloon connect a pathway for blood through the shaft 9 of the perfusion balloon catheter to the distal end of the catheter 17. The perfusion fluid channel 15 connects to the holes at the proximal 16 and distal end 17 of the balloon. The perfusion holes 16, 17 are penetrating through the membrane 12, 13 carrying the liquid oxygen carrier. Thus, blood perfusion through the balloon carries blood that is oxygenated by the membrane at the proximal end of the inflated balloon and is oxygenated beyond the distal end of the inflated balloon by the membrane after passage through the balloon. The guide-wire for advancing the perfusion balloon is provided at its distal part 28 with the oxygen delivery source, which is impregnated with the liquid oxygen carrier, preferably in the form of a liquid oxygen solution. A stent 29 can be mounted onto the balloon surface.

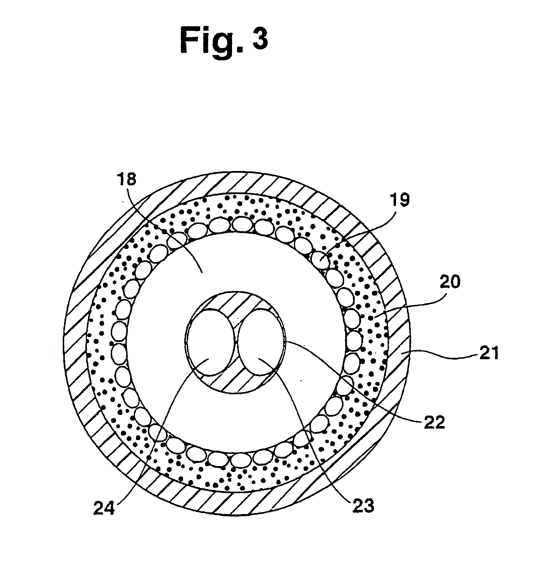

Figure 3 schematic cross-sectional view of a medical device containing a liquid oxygen delivery source being encompassed by a housing sealing off the impregnated source in accordance with the present invention. The oxygen delivery source such as a tube with multiple channels 18, 23, 24 with an attached thin film membrane 19 incorporating the oxygen carrier is placed in an container 21 filled with a liquid oxygen carrier solution 20. The container eliminates any dissipation of liquid or gas, and is sought to be a storage place for the oxygen delivery source. One channel 18 may serve for guiding warm or cold fluids for altering release of the oxygen carrier from the membrane 19, the others lumens 23, 24 may serve for guiding blood, therapeutic fluids or guide-wires.

## Claims

1. Medical device for temporary or permanent insertion into a blood vessel comprising a delivery source of oxygen, wherein the delivery source comprises a porous substrate, **characterized in that** the porous substrate has at least one liquid oxygen carrier incorporated therein by impregnating the porous substrate at the operating table or by pre-impregnating the porous substrate with liquid oxygen carrier, wherein the oxygen carrier is an oxygenated fluorocarbon solution, which is released from the pores.

2. Medical device of claim 1, wherein said substrate forms at least one membrane or a thin film layer.

3. Medical device of claim 1 or 2, wherein said substrate is at least a part of a hollow tube, a perfusion balloon catheter, a bypass graft, an endovascular stent inserted temporarily or permanently into or at a blood vessel, a transvascular implant directly connected to blood vessels.

4. Medical device of any of claims 1 through 3, wherein said substrate comprises a porous polymer, preferably with a pore size in the range of 20 - 200 microns.

5. Medical device of claim 4, wherein the polymer of said substrate is selected from a group of polymers such as teflon, polyethylene, polyethylene terephtalate, polyphosphazene, nylon, silicon, and cellulose actetate.

6. Medical device of any of claims 1 through 5, wherein the delivery source is a plurality of interconnected tubes whereas the inner surface of an inner tube is a porous membrane and incorporates liquid oxygen carrier, and the outside of the inner tube is a channel transport system for cold and warm liquids formed by the other tubes of the plurality of tubes.

7. Medical device of any of claims 1 through 6, wherein said oxygen carrier is a pre-oxygenated fluorocarbon solution.

8. Medical device of any of the previous claims, wherein said substrate incorporating the pre-oxygenated oxygen carrier is sealed within a protective means such as a removable film coating, a plastic sheath, metal or glass container to prevent dissipation of liquid and gas.

## Patentansprüche

1. Medizinische Vorrichtung zum temporären oder permanenten Einführen in ein Blutgefäß umfassend eine Sauerstoffabgabequelle, wobei die Sauerstoffabgabequelle ein poröses Substrat umfasst, **dadurch gekennzeichnet, dass** in dem porösen Substrat durch Imprägnieren des porösen Substrates am Operationstisch oder durch Vorimprägnierung des porösen Substrates mit flüssigem Sauerstoffträger zumindest ein flüssiger Sauerstoffträger beinhaltet ist, wobei der Sauerstoffträger eine oxygenierte Fluorkarbonlösung ist, die von den Poren freigegeben wird.

2. Medizinische Vorrichtung nach Anspruch 1, wobei das Substrat zumindest eine Membran oder eine Dünnfilm-Schicht bildet.

3. Medizinische Vorrichtung nach einem der Ansprüche 1 oder 2, wobei das Substrat zumindest ein Teil eines hohlen Schlauches, eines Perfusions-Ballonkatheters, ein Bypass-Transplantat, ein endovaskularer Stent, der temporär oder permanent in das oder an das Blutgefäß eingebracht ist, ein transvaskulares Implantat, das direkt mit Blutgefäßen verbunden ist, ist.

4. Medizinische Vorrichtung nach einem der Ansprüche 1 bis 3, wobei das Substrat ein poröses Polymer, vorzugsweise mit einer Porengröße im Bereich von 20 - 200 µm, umfasst.

5. Medizinische Vorrichtung nach Anspruch 4, wobei das Polymer des Substrates ausgewählt ist aus einer Gruppe von Polymeren, wie Teflon, Polyethylen, Terephthalat, Polyphosphazen, Nylon, Silikon und Zellulose-Azetat.

6. Medizinische Vorrichtung nach einem der Ansprüche 1 bis 5, wobei die Abgabequelle eine Vielzahl miteinander verbundener Schläuche ist, wobei die innere Oberfläche eines inneren Schlauches eine poröse Membran ist und flüssigen Sauerstoffträger beinhaltet und die Außenseite des inneren Schlauches ein Kanaltransportsystem für kalte und warme Flüssigkeiten ist, die durch die anderen Schläuche der Vielzahl von Schläuchen gebildet ist.

7. Medizinische Vorrichtung nach einem der Ansprüche 1 bis 6, wobei der Sauerstoffträger eine voroxygenierte Fluorkarbon-Lösung ist.

8. Medizinische Vorrichtung nach einem der vorstehenden Ansprüche, wobei das Substrat, das den voroxygenierten Sauerstoffträger beinhaltet, mit Schutzmitteln, wie einer entfernbaren Filmbeschichtung, einer Plastikhülle, Metall- oder Glassbehälter abgedichtet ist, um ein Entweichen von Flüssigkeiten und Gas zu verhindern.

## Revendications

1. Dispositif médical d'insertion temporaire ou permanente dans un vaisseau sanguin, comprenant une source de distribution d'oxygène, la source de distribution comprenant un substrat poreux, **caractérisé en ce que** le substrat poreux a au moins un véhicule d'oxygène liquide incorporé dans celui-ci par imprégnation du substrat poreux à la table d'opération ou par pré-imprégnation du substrat poreux avec un véhicule d'oxygène liquide, le véhicule d'oxygène étant une solution de fluorocarbure oxygénée qui est libérée des pores.

2. Dispositif médical selon la revendication 1, dans lequel ledit substrat forme au moins une membrane ou une couche de film fin.

3. Dispositif médical selon la revendication 1 ou 2, dans lequel ledit substrat est au moins une partie d'un tube creux, un cathéter de perfusion à ballonnet, un greffon de pontage, un stent endovasculaire inséré temporairement ou de façon permanente dans ou au niveau d'un vaisseau sanguin, un implant transvasculaire relié directement aux vaisseaux sanguins.

4. Dispositif médical selon l'une quelconque des revendications 1 à 3, dans lequel ledit substrat comprend un polymère poreux, de préférence présentant une taille de pores de l'ordre de 20 à 200 microns.

5. Dispositif médical selon la revendication 4, dans lequel le polymère dudit substrat est choisi dans un groupe de polymères tels que le téflon, le polyéthylène, le polyéthylène téréphtalate, le polyphosphazène, le nylon, le silicium et l'acétate de cellulose.

6. Dispositif médical selon les revendications 1 à 5, dans lequel la source de distribution est une pluralité de tubes interconnectés alors que la surface interne d'un tube interne est une membrane poreuse et comprend un véhicule d'oxygène liquide et l'extérieur du tube interne est un système de transport canalaire de liquides froids et chauds formé par les autres tubes de la pluralité de tubes.

7. Dispositif médical selon l'une quelconque des revendications 1 à 6, dans lequel ledit véhicule d'oxygène est une solution de fluorocarbure pré-oxygénée.

8. Dispositif médical selon l'une quelconque des revendications précédentes, dans lequel ledit substrat comprenant le véhicule d'oxygène pré-oxygéné est scellé dans un moyen protecteur tel qu'un revêtement de film amovible, une gaine de plastique, un récipient en métal ou en verre pour empêcher la dissémination de liquide et de gaz.
